# EUROPEAN PATENT SPECIFICATION

(11) **EP 2 136 698 B1**
(45) Date of publication and mention of the grant of the patent: **21.07.2010**
(21) Application number: 07736763.9
(22) Date of filing: 04.04.2007
(51) Int. Cl.: A61B 1/05

(54) **TELEOPERATED ENDOSCOPIC CAPSULE**
FERNBEDIENTE ENDOSKOPISCHE KAPSEL
CAPSULE ENDOSCOPIQUE TÉLÉCOMMANDÉE

(43) Date of publication of application: 30.12.2009
(73) Proprietor: Scuola Superiore di Studi Universitari e di Perfezionamento Sant'Anna, 56127 Pisa (IT); Korea Institute of Science and Technology, Seoul 136-791 (KR)
(72) Inventor: QUIRINI, Marco, I-51015 Monsummano Terme (Pistoia) (IT); WEBSTER, Robert, J., III, Nashville, TN 37204 (US); MENCIASSI, Arianna, I-56025 Pontedera (Pisa) (IT); DARIO, Paolo, I-57100 Livorno (IT)
(74) Representative: Bardini, Marco Luigi
(86) International application number: PCT/IT2007/000259
(87) International publication number: WO 2008/122997

(56) References cited:
- WO-A-2005/082248
- WO-A-2006/121239
- US-A1- 2002 171 385
- US-A1- 2002 173 700
- ARIANNA MENCIASSI, MARCO QUIRINI, PAOLO DARIO: "Microrobotic Technologies in Endoluminal Applications"[Online] October 2006 (2006-10), XP002460281 Paris, France Retrieved from the Internet: URL:http://iarp06.robot.jussieu.fr/Papers/ Menciassi/iarp%20-%20Presentation%20-%20Me nciassi.pdf> [retrieved on 2007-11-28]
- MENCIASSI A ET AL: "Towards active capsular endoscopy: preliminary results on a legged platform" CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748) IEEE PISCATAWAY, NJ, USA, 2006, page 4 pp., XP002460282 ISBN: 1-4244-0032-5

## Description

### Field of the invention

The present invention relates generally to the field of endoscopic devices and more precisely, relates to an endoscopic capsule for diagnostic and/or therapeutic purposes, remote controlled, and able to move inside various areas in the human body, and in particular in the gastrointestinal area, more in particular, in a passive manner in the small intestine and in an active manner in the colon.

### Description of the prior art

As is known in recent years there has been an increasing interest in devices that permit endoscopic examination and treatment in the most autonomous and least invasive manner.

For this purpose semi-automatic locomotive solutions have been studied with terminals mounted with a video camera, based on a "inchworm locomotion model, such as the endoscopic device described in WO02/68035. These systems have the drawback of limited possibility of control of the locomotion parameter, and the speed cannot be varied at all. In addition, they also have the drawback that their bodies creep along the walls of the body cavity inside which they move without being able to avoid possible lesions and pathological sites.

Endoscopic devices controlled from the exterior through force fields (a magnetic field for example) have been disclosed; these devices require that the patient wear suitable equipment to generate the field). As an example, the device known as Norika 3 produced by the Japanese company RF System Lab can be taken as a reference. However the use of these devices can create problems and even be dangerous because of possible interference with other biomedical devices applied to the patient. Moreover, this kind of externally controlled endoscopic devices also involve the risk of side effects due to extensive exposure to electromagnetic fields.

A completely autonomous endoscopic device for detecting images with wireless data transmission, integrated in a small capsule, is described in the US patent no.5604531. The device comprises a CMOS image generator, a transmitter, LED illumination and energy supply provided by a watch battery. The main limits of this device concern the lack of active locomotion control: the capsule move forward through the effect of normal peristalsis and, during its movement, cannot stopped nor oriented.

Some solutions intended to provide a solution to the problem of the active locomotion control comprise a capsule equipped with autonomous locomotion means controllable by an external operator. In general, these solutions comprise a cylindrical body having a plurality of locomotion modules arranged on the surface that permit the movement inside the body cavity to be explored. Housed in the cylindrical body are a source of energy, a microcontroller to drive the locomotion modules according to commands transmitted through remote control by the operator, a video camera for image acquisition controlled by a micro-controller, a transceiver system to receive the commands transmitted through remote control by the operator and to transmit the acquired images through the video camera.

In any case, these "autonomous" systems require that a gas be introduced to distend the walls of the cavity to be explored so as to permit the movement of the capsule, and an optimised vision of the cavity. The use of this gas is a painful experience for the patient subjected to endoscopic examination.

A solution aimed at avoiding the use of gas is described in WO2006121239 relating to a capsule with integrated locomotion means and a radio control managed by an external operator. The capsule has an external cylindrical body provided with a transparent spherical cap at the end housing a video camera and various electronic means for operation and control as well as for radio signal transmission/reception. The capsule locomotion means are basically composed of hooking teeth that protrude from longitudinal slits formed on the cylindrical body and that are hinged to a block mounted on a nut screw cursor coupled with a worm screw driven by an electric motor present inside, and firmly attached to, the body. When the worm screw is rotated in one direction, the teeth extend outside the body to grip the walls of the cavity to go through and to be examined, and the block with the teeth moves towards the rear end of the body; however, since the teeth are hooked into the cavity walls, this results in a forward movement of the body in the cavity similar to a creeping movement. When the worm screw is turned in the opposite direction, the block performs a forward axial movement, while the teeth are retracted back inside the body and the capsule remains still.

However fully satisfactory the component miniaturisation may be, the capsule in this solution is unable to turn or tilt to observe details of the cavity under examination. Moreover, it is unable to perform a reverse movement nor can it move in vertical direction, in view of the fact that for a part of the locomotion cycle, none of the teeth are able to hook into the cavity wall. Moreover, the capsule is unable to adapt itself to the various geometries featuring the gastrointestinal tract and it does not comprises any systems able to distend the surrounding intestinal walls adequately, an aspect which is very important from a diagnostic viewpoint to permit optimal observation of intestinal tissue.

Another solution aimed at avoiding the use of gas, is disclosed in WO2005082248. In particular, in this case the locomotion modules are formed by six legs hinged to the cylindrical body and controlled by a drive system composed of wires connected to each leg and acting in opposition to displace it angularly around the hinge axis. The wires are connected to electric contacts by transmission means. Both the legs and the wires are made in SMA (Shaped Memory Alloy). One of the two opposite wires is heated through the passage of electrical current, bringing it to the phase transition temperature of the SMA, resulting in the contraction of the wire (the cold wire is deformed through the action of the hot wire) and the rotation of the leg. When the electrical supply is cut off, the temperature is lowered and the wire stops its traction force, permitting the counteracting wire, heated successively, to contract, thus completing the return movement of the leg and at the same time, bringing the first wire back to its original length. The legs project in a radial direction in relation to the axis of the cylindrical body, and in an equally distanced manner around said axis, so that when the cavity tracts to be explored have a cross-section smaller than the size of the capsule with the legs projected, it provokes the expansion of the cavity.

However advantageous this solution may be as regards the possibility of directing the capsule, the compact size and the "distension" of the tracts to be explored compared to the prior art capsule devices, it nevertheless possesses certain aspects to be improved; these aspects are mainly related to the high power consumption due to the wire heating, this resulting in the reduction in the operating range of the device. Moreover, the force generated by the SMA wires can be insufficient for the complete expansion of the intestinal lumen, this resulting in a difficult forward movement due to the friction provided by the non-distended tissue. Furthermore, not always the legs are able to remain attached to, or in adhesion with, the cavity walls because of the wall irregularity and slippery surface. To ensure adherence, the force of contact must be increased, but this requires the use of wires with a larger section, resulting in a size increase and greater energy consumption, as well as a reduction in the frequency of the locomotion cycle because of the amount of heat to be eliminated.

In US 2002/173700 a capsule type micro-robot system is disclosed.

The main object of the present invention is to provide an endoscopic capsule having autonomous movement and energy supply within the body cavity, with the possibility of controlling the movement from the exterior to permit medical, diagnostic and therapeutic procedures, to be carried out and in particular, to be able to transmit images of the interesting areas in the body cavity through which it passes.

Another object of the present invention is to provide a teleoperated endoscopic capsule able to move in an autonomous manner inside the cavity to be explored without the need for using gas to expand the cavity walls.

Another object of the present invention is to provide a teleoperated endoscopic capsule that can adapt well to the environment in which it is placed without provoking irritation or injury to the surrounding tissues.

A further object of the invention is to provide an endoscopic capsule equipped with autonomous locomotion means, wherein its movement can be easily stopped, accelerated or reduced according to need through an external remote control.

Another object of the invention is to provide an endoscopic capsule equipped with autonomous locomotion means that is able to turn corners easily.

### Summary of the Invention

These objects are achieved with a teleoperated endoscopic capsule for diagnostic and/or therapeutic purposes inside cavities in the human body, comprising a body defining a front part and a rear part, locomotion legs able to extend from said body, and moving means for said legs housed within the body, an energy source, means for acquiring images, means for signal reception/transmission from and to an operator in order to permit capsule control and the transfer of the acquired images. The legs are hinged to said body and are divided into two separate groups, the moving means comprising two driving devices, each one comprising a motor connected to a corresponding worm screw on which a translatable nut screw cursor is engaged, said nut screw cursor being kinematically connected to the legs of a respective group of legs..

### Brief description of the drawings

Further characteristics and advantages of the endoscopic capsule according to the present invention will be made apparent from the following description of its embodiment, provided as a non-limiting example with reference to the appended drawings wherein:

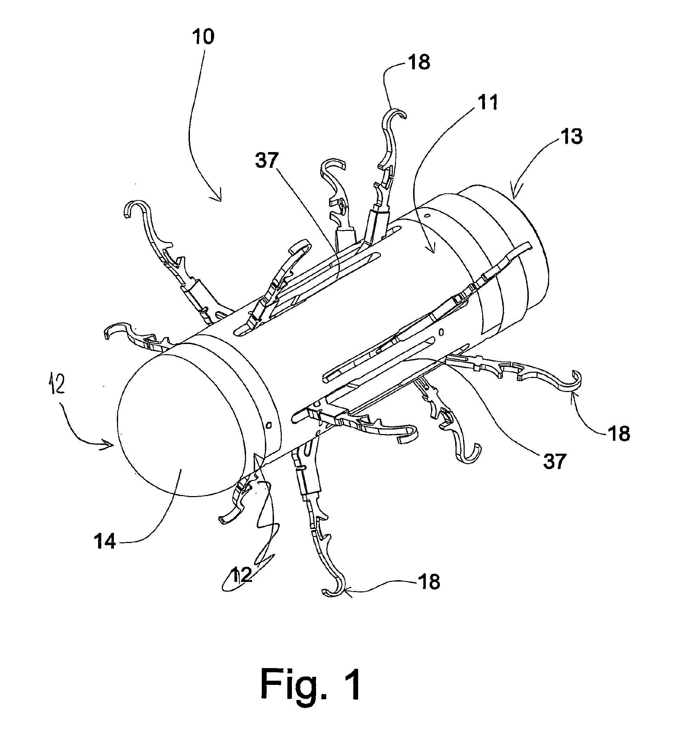

- Figure 1 shows an axonometric view of a capsule according to the invention, illustrating the locomotion legs in their most extended position;
- Figure 2 shows an axonometric view of the capsule of figure 1, partially in section;
- Figure 3 shows an axonometric view of internal components of the capsule in the previous figures, in particular the device for the movement of the legs;
- Figure 4 shows a front view of the capsule, on a plane perpendicular to the axis of the capsule body;
- Figure 5 shows a schematic side view of part of the device which drives the leg movement, in which the most outspread position of a leg and the least outspread position shown in dotted line are depicted;
- Figure 6 shows a schematic side view of a part of the leg movement driving device, partially in section;
- Figure 7 shows a front view of a part of the driving device.

### Detailed description of the invention

With reference to the aforesaid figures, a teleoperated endoscopic capsule for diagnostic and/or therapeutic purposes according to the invention is identified throughout by the numeral 10.

The capsule 10 comprises a body 11, having a substantially cylindrical shape that defines a front part 12 and a rear part 13.

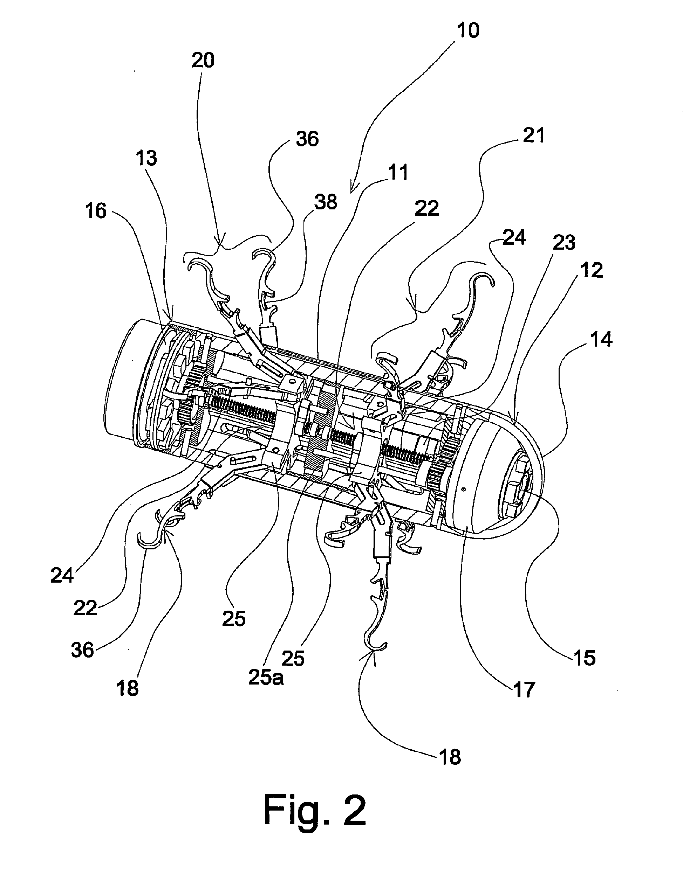

At the end of the front part 12 the body 11 is provided with a transparent dome 14, inside which are arranged means for image acquisition 15, such as a digital video camera (associated lighting means of the known type, not shown) connected to a source of electrical energy 16 such as a watch battery, for example; both the video camera and the battery are shown schematically in figure 2.

Means for signal reception/transmission 17 to and from the external operator are housed under the dome 14, to permit remote control of the capsule and the transfer of the acquired images to an external terminal, with which the operator is able to interface. These means are substantially of the known type, such as those described in the international patent application WO2005082248, but other functionally equivalent means can be used in alternative.

It is obvious how, in other embodiments, the video cameras can be more than one in number, such as two for example, positioned in the front part 12 and rear part 13 of body 11 respectively.

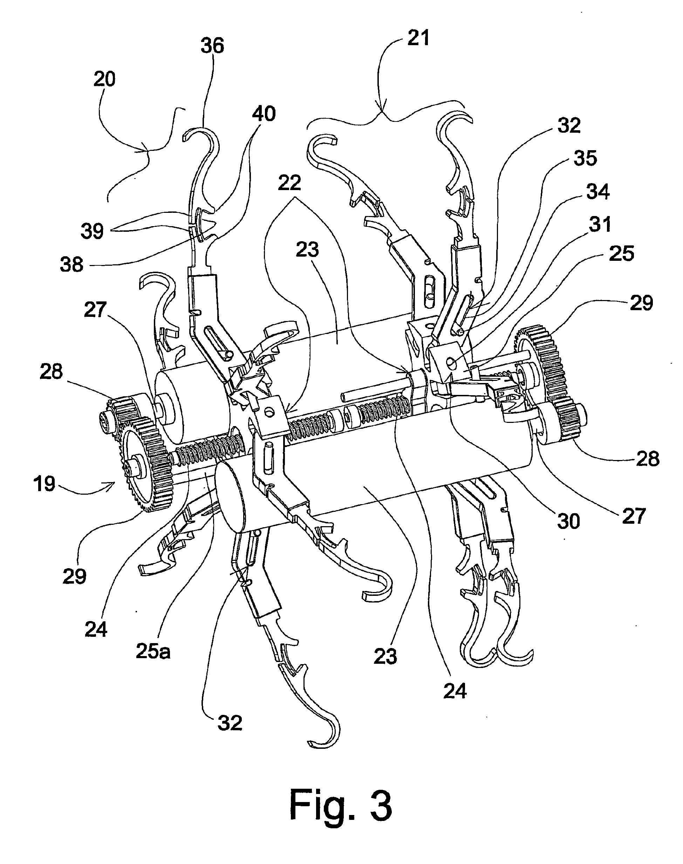

The capsule 10 also comprises locomotion legs 18 able to project from the body 11; the means 19 for moving the legs 18, illustrated in particular in figure 3, are positioned inside body 11. In particular, the legs 18 are divided into two separate groups; a first group 20 is positioned towards the front part 12 the body 11, while a second group 21 is positioned towards the rear part 13.

Figures 2 and 3 show how the moving means 19 comprise two separate driving devices 22 for the legs 18, which operate on the first and second groups 20 and 21 respectively; preferably, said driving devices 22 are substantially of the same type, as described below.

Each driving device 22 comprises a motor 23 connected to a corresponding worm screw 24 engaged with a nut screw cursor 25 kinematically connected to the legs 18. The nut screw cursor 25 is forced to translate along the same worm screw 24 because of the slidable coupling to a guide bar 25a, parallel to the worm screw 24 and connected rigidly to body 11.

For example motor 23 is a direct current brushless type electric motor produced by Namiki Precision Jewel Co., Ltd., having an external diameter of 4mm and a total length of 16.2 mm; said motor has an integrated speed reducer and the maximum delivered torque at the speed reducer shaft is equal to 2.92 mNm (the same motor without the speed reducer has a maximum delivered torque at the shaft equal to 0.058 mNm); said electric motor is powered by the battery 16.

In particular, according to an important characteristic of the invention, the worm screws 24 of the two driving devices 22 are coaxial with the axis of body 11 and the motors 23 are positioned with their drive shafts 27 parallel to the axis of body 11, and therefore on the opposite sides with respect to the common axis of the worm screws 24.

Each motor 23 is connected to the corresponding worm screw 24 by means of a standard gearing with a transmission ratio less than 1; in particular the standard gearing is formed by a pinion 28 fixed at the end of the drive shaft 27 of motor 23 and a gearwheel 29, having a diameter larger than the pinion 28, fixed at the end of the worm screw 24. The transmission ratio ranges between 0.420 and 0.430, preferably equal to 0.425; this is an optimal value in terms of the force delivered to the legs with the space occupied by the standard gearing. The motors 23 are positioned opposite to each other, and therefore the gearwheels 29 of the two worm screws 24 are positioned at each end of the screws.

The moving means 19 of the legs 18 also comprise electronic means (for simplicity not numbered in the figures) substantially of the known type, such as those described in the aforesaid international patent application W02005082248, able to control the independent motion of the two groups of legs 18, according to the commands from the external controller.

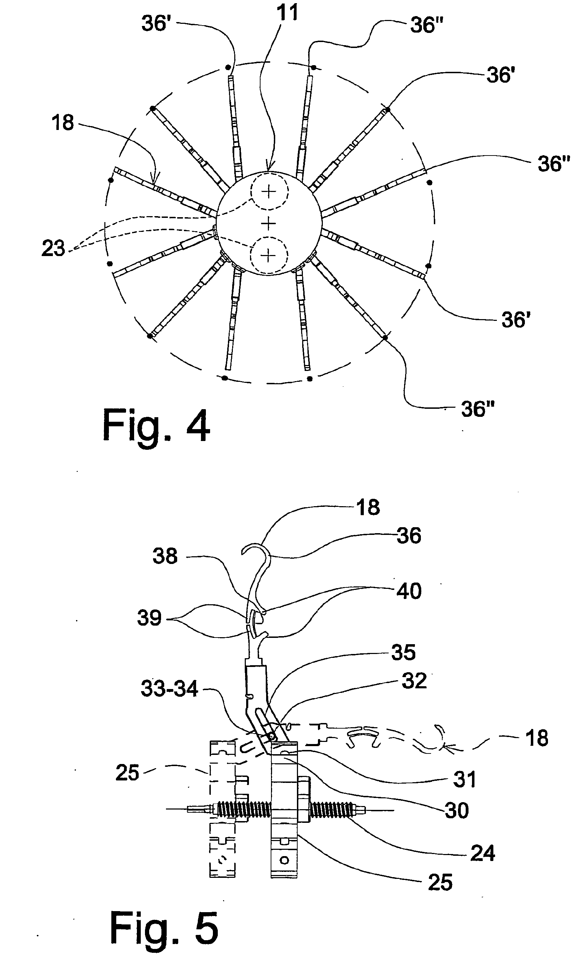

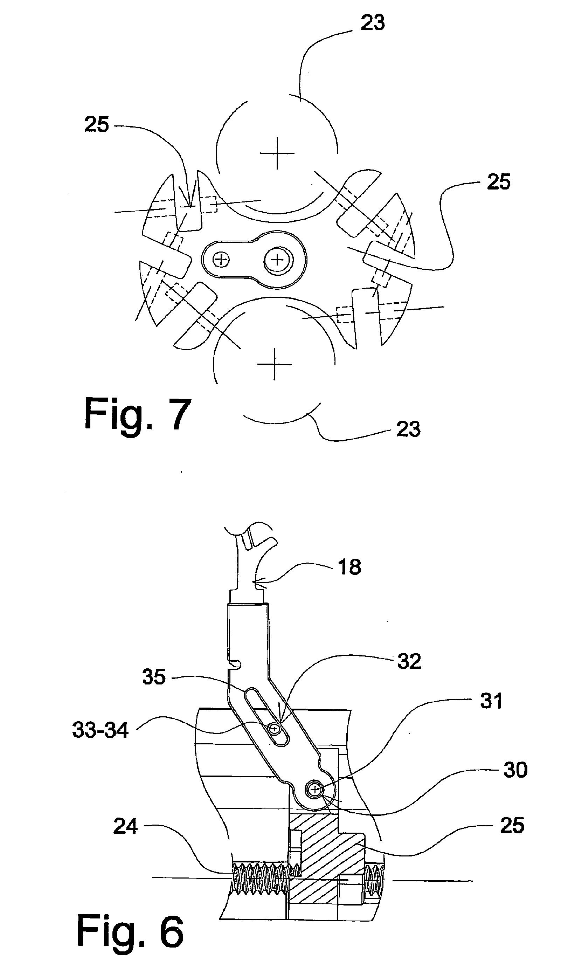

Each group 20 and 21 of legs 18 is connected to a respective nut screw cursor 25 (see in particular figures 5, 6 and 7). Preferably, at a first end of each of its own legs 18, each group 20 and 21 has a first restraint 30 composed of a rotational attaching hinge 31 fixed to the nut screw cursor 25, and at an intermediate position of the sam leg 18, a second restraint 32 that allows a roto-translation, with respect to a fixed point 33 of the body 11, the rotation axis being parallel to the axis of the hinge 31.

In particular, said second restraint 32 comprises a rotation pin fixed to the body 11 and a guide groove 35 defined along the corresponding leg 18; said pin 34 and groove 35 are coupled together with relative sliding, to allow the aforesaid rototranslation to be performed.

In practice, each nut screw cursor 25 can translate along the relative worm screw 24 from an initial position, in which the corresponding legs 18 are in their position of minimum extension (as can be seen in the part marked with the dotted line shown in figure 5), laid along body 11, to a final position, in which the legs 18 are in their maximum extension, projected towards the exterior of the body 11 with a angle such that the free ends 36 of the legs are positioned at the maximum distance from the axis of the body 11 (the part marked with the dark line in the drawing in figure 5).

The driving devices 22 are housed inside the body 11, which is formed with longitudinal slots 37 on the external surface, passing from the interior towards the exterior, to permit the legs 18 to protrude. More in particular, on the body 11 there are provided two groups of angularly equispaced. slots, one group for each group of legs, extended respectively from the front end and from the rear end of the body 11 as far as an intermediate position thereof. The slots of one group are not aligned with the corresponding slots of the other group, but are staggered two by two, even though they are positioned closely to one another.

Advantageously, each group of legs 20 and 21 is formed of six legs 18. This choice is the result of a number of tests performed with capsule prototypes presenting different numbers of legs. It was possible to observe from these tests that the locomotion performance increases with an increase in the number of legs. This is due to the fact that with an increased number of legs, the force of propulsion as well as the distension of the intestinal wall is distributed in a much more uniform manner. Therefore, while complying with the dimensional limits imposed by the diameter of the endoscopic capsules available on the market (approximately 11mm), the number of legs has been maximised as far as twelve in number (six for each group). This number of legs achieves the aims proposed, as will be described more clearly below.

In figure 4 the free ends of the legs of the first group 20 are identified with the numeral 36', while the free ends of the legs of the second group 21 are identified with the numeral 36". As shown in figure 4, the projections of the free ends 36 of the legs 18 of both the first group 20 and the second group 21 on a plane orthogonal to the axis of the corresponding worm screws 24 (coinciding with the axis of body 11) are arranged substantially on a same circumference, shown by the dotted line, whose centre coincides with said axis.

As can be seen from the figure, the free ends 36 are set at substantially the same angular distance from each other along the circumference. In particular, as shown in figure 4, when the legs 18 of both groups 20-21 are in their outspread position, the projections of the free ends on the plane orthogonal to the axis of the corresponding worm screw 24, are arranged on a same circumference. More precisely, the legs belonging to the two groups alternate in the projection on the plane orthogonal to the axis of the capsule and, for dimensional reasons, the two legs of each group farthest from the respective motor are slightly displaced on an angle (about 4°) in relation to their ideal position. The above arrangement is required to avoid the degeneration of the volume of the capsule body that accounts for the equal distance spacing of the legs. More precisely, should the longitudinal slots of the aforesaid legs (slots inside which the said legs house when they fold to their completely retracted position) be arranged at the same angular distance in relation to one another, this would result in a collision between the legs during the retraction stage. This interference condition has been avoided by spacing the legs at a slight distance from each other.

In an intermediate point between the free end 36 and the guide groove 35, each leg 18 is formed with an elastic knee portion 38 that forms a further degree of freedom to adapt the leg to the yielding nature of the tissue with which it comes into contact; in other words the terminal portion of the leg is elastically flexible around the knee portion.

Two opposing extensions 39 are positioned near the knee portion 38, to limit the leg 18 rotation for a few degrees in its extension direction, while another pair of extensions 40 can be positioned at the opposite side of the leg 18 to abut each other after a wide rotation around the knee 38. The pair of extensions 40 therefore limits the amount of flexion to which the leg 18 could be subjected, in order to prevent any possible damage thereof.

As shown in the figures, the free end of each leg is substantially hook-shaped for gripping the mucous membrane of the intestine to permit the forward movement; the size of the hooks is smaller than the thickness of the mucous membrane of the intestine (0.2 mm), in this way they do not damage the underlying tissue.

The function of the two groups of legs is different. The second group 21 is mainly aimed at driving the capsule motion, while the first group 20 is mainly aimed at attaching the capsule to the walls of the cavity under exploration, to facilitate the curved trajectories and to distend the walls to provide an optimal view thereof.

The endoscopic capsule according to the invention can be advantageously coated with a biocompatible and biodegradable layer that prevents the accidental outward extension of the legs during ingestion, making the swallowing action easier and safer. When the capsule reaches the stomach, the coating is destroyed by the acidity of the environment thus permitting the leg movements.

This capsule structure makes it possible to respect important constructive and dynamic parameters such as compact size (a length preferably between 24 mm and 28 mm), widespread angles for the legs (preferably between 90° and 130°), internal size that is sufficient to house the electronic components (thanks to motors that do not occupy more than 10,5% of the total volume of the body 11) controlled strength at the free ends of the legs (preferably between 1.8N and 3.2N) and number of legs (between 8 and 12).

Furthermore, thanks to the structure of the capsule according to the invention, it is possible to use a number of legs (twelve, in the example described) that is much larger than in other prior art capsules.

This provides several advantages, among which: a) the possibility of distending the cavity walls more easily for exploration, whereby the use of gas to expand the cavity is avoided; b) the possibility of low interaction force of each leg with the cavity walls, but with an overall force strong enough to permit hook gripping and locomotion along the walls, with the obvious advantage concerning the risk of tissue irritation and damage, and; c) greater flexibility in adjusting the locomotion speed.

The capsule according to the invention can be modified and varied in several ways, all of which are within the scope of the invention; all the details may further be replaced with other technically equivalent elements. In practice, the materials used, so long as they are compatible with the specific use, as well as the dimensions, may be any according to the requirements and the state of the art.

Wherever the characteristics and techniques described in any claim are followed by a specific reference, these have been included as an example for the sole purpose of making the claim descriptions easier to understand, and therefore they impose no limits on the interpretation of the element they refer to.

## Claims

1. Teleoperated endoscopic capsule for diagnostic and/or therapeutic purposes inside a cavity in a human body, comprising a body (11) having a front part (12) and a rear part (13), locomotion legs (18) able to project from said body (11) and moving means (19) for said legs (18) housed within the same body (11) a source of energy (16), means for image acquisition (15), means for reception/transmission of signals (17) from and to an operator for permitting the control of the capsule and transfer of acquired images, wherein said legs (18) are hinged to said body (11) and are subdivided into two separate groups (20, 21), **characterised in that** said moving means (19) comprising two driving devices (22) each one comprising a motor (23) connected to a corresponding worm screw (24) on which a translatable nut screw cursor (25) is engaged, said nut screw being kinematically connected to the legs (18) of a respective group (20, 21).

2. Teleoperated endoscopic capsule, according to claim 1, **characterised in that** said at least one first group (20) comprises, at a first end of each leg (18), a first restraint (30) formed by a hinge, for pivotally fixing the leg to the relevant nut screw cursor (25) and, in an intermediate position of the same leg (18), a second restraint (32) for permitting a roto-translation, with the rotation axis parallel to the axis of said hinge (31), with respect to a fixed point (33) of said body (11).

3. Teleoperated endoscopic capsule, according to claim 2, **characterised in that** said second restraint (32) comprises a rotation pin (34) fixed to said body (11) and a guide groove (35) formed along said leg (18), said pin (34) and said groove (35) being coupled with relative sliding.

4. Teleoperated endoscopic capsule according to any one of the previous claims, **characterised in that** it comprises a first group (20) of legs (18) arranged near the front part (12) of said body (11) and a second group (21) of legs (18) positioned near the rear part (13) of said body (11).

5. Teleoperated endoscopic capsule according to any one of the previous claims, **characterised in that** the worm screws (24) of said driving devices (22) are coaxial.

6. Teleoperated endoscopic capsule according to any one of the previous claims, **characterised in that** each motor (23) is connected to the corresponding worm screw (24) by means of a gearing having a transmission ratio less than 1.

7. Teleoperated endoscopic capsule according to claims 4, 5 or 6, **characterised in that** said gearing comprises a first gearwheel (28) fixed at the end of the drive shaft (27) of said motor (23) and a second gearwheel (29), having a diameter larger than said first gearwheel (28), fixed at the end of said worm screw (24), said two motors (23) of said two driving devices (22) of the two groups (20, 21) of legs (18) being positioned at opposite sides of said worm screws (24).

8. Teleoperated endoscopic capsule according to claim 7, **characterised in that** the transmission ratio of said gearing is between 0.420 and 0.430.

9. Teleoperated endoscopic capsule according to any one of the previous claims, **characterised in that** said body (11) is formed with longitudinal through slots (37) on the external surface for housing said legs (18) therein.

10. Teleoperated endoscopic capsule according to claim 9, **characterised in that** two groups of angularly equidistant slots (37), one for each group (20, 21) of legs (18), extend respectively from the front end and from the rear end of said body (11) as far as an intermediate position, the slots of one group and the slots of the other group being set in two by two staggered positions.

11. Teleoperated endoscopic capsule according to any one of the previous claims, **characterised in that** the front part (12) of said body (11) is equipped with a transparent dome (14) inside which the image acquisition means (15) are mounted, in particular, a video camera.

12. Teleoperated endoscopic capsule according to any one of the previous claims, **characterised in that** said at least one motor (23) is a direct current, brushless electric motor, having an external diameter comprised between 3.5 mm and 4.5 mm and a total length comprised between 15.2 mm and 17.2 mm, said motor being equipped with a speed reducer, the maximum torque delivered to the shaft of said speed reducer being equal to approximately 2.92 mNm.

13. Teleoperated endoscopic capsule according to any one of the previous claims, **characterised in that** each group (20, 21) of legs (18) is formed by a number of legs (18) between four and six, the projections of the free ends (38) of said legs (18) on a plane orthogonal to the axis of said worm screws (24) lying substantially on a same circumference whose centre coincides with said axis, said free ends (36) being substantially equidistant from each other along said circumference, said moving means (19) permitting the arrangement of said legs (18) from a maximum outspread extension where said legs (18) project outwards the exterior of said body (11) to a minimum outspread extension where said legs (18) are positioned along said body (11).

14. Teleoperated endoscopic capsule according to claim 13, **characterised in that** the said legs (18) of both groups (20, 21) are positioned in the maximum outspread extension, the projections of the free ends (36', 36") of different groups (20, 21) on the plane orthogonal to the axis of the corresponding worm screws (24) are alternated and substantially equidistant along a same circumference.

15. Teleoperated endoscopic capsule according to any one of the claims from 3 to 14, **characterised in that** each leg (18) presents, in an intermediate point between said free end (36) and said guide groove (35), an elastic knee portion (38) that provides a further degree of freedom to adapt the leg to the yielding nature of the tissue with which it makes contact.

16. Teleoperated endoscopic capsule according to claim 15, **characterised in that** two opposing extensions (39) are provided at said knee portion (38) for abutting with each other to limit the rotation of said leg (18) in the direction of its extension.

17. Teleoperated endoscopic capsule according to claim 15 or 16, **characterised in that** a further pair of extensions (40) are provided at said knee portion (38) for abutting with one another after a wide rotation around said knee portion (38).

18. Teleoperated endoscopic capsule according to any one of the previous claims, **characterised in that** the free end (36) of each leg (18) has a hook shape.

19. Teleoperated endoscopic capsule according to any one of the previous claims, **characterised in that** it has a overall length substantially comprised between 24 mm and 28 mm, opening angle of the legs substantially comprised between 90° and 130°, electric motors that occupy no more than 10.5% of the total volume of the body 11, and force generated at the free ends (36) of each the legs (18) substantially between 1.8N and 3.2N.

## Patentansprüche

1. Fernbediente endoskopische Kapsel für diagnostische und/oder therapeutische Zwecke innerhalb eines Hohlraums in einem menschlichen Körper, enthaltend einen Körper (11) mit einem Vorderteil (12) und einem Hinterteil (13), Fortbewegungsbeine (18), die in der Lage sind, von dem Körper (11) vorzustehen, und für die Beine (18) Bewegungseinrichtungen (19), die innerhalb desselben Körpers (11) untergebracht sind, eine Energiequelle (16), Einrichtungen zur Bilderfassung (15), Einrichtungen zum Empfang/zur Übertragung von Signalen (17) von und zu einem Operator, um die Steuerung der Kapsel und die Übertragung von erfassten Bildern zu gestatten, wobei die Beine (18) an dem Körper (11) angelenkt und in zwei separate Gruppen unterteilt sind, **dadurch gekennzeichnet, dass** die Bewegungseinrichtungen (19) zwei Antriebsvorrichtungen (22) enthalten, von denen jede einen Motor (23) enthält, der mit einer entsprechenden Schneckenwelle (24) verbunden ist, an welcher ein umsetzbarer Spindelmuttercursor (25) in Eingriff ist, welche Spindelmutter kinematisch mit den Beinen (18) einer entsprechenden Gruppe (20, 21) verbunden ist.

2. Fernbediente endoskopische Kapsel nach Anspruch 1, **dadurch gekennzeichnet, dass** die wenigstens eine erste Gruppe (20) an einem ersten Ende jedes Beins (18) eine erste Begrenzung (30), die durch ein Gelenk gebildet ist, um das Bein drehbar an dem entsprechenden Schraubspindelcursor (25) zu befestigen, und in einer Zwischenposition desselben Beins (18) eine zweite Begrenzung (32) enthält, um eine Roto-translation mit der Drehachse parallel zu der Achse des Gelenks (31) bezüglich einem festen Punkt (33) des Körpers (11) zu gestatten.

3. Fernbediente endoskopische Kapsel nach Anspruch 2, **dadurch gekennzeichnet, dass** die zweite Begrenzung (32) einen Drehstift (34), der an dem Körper (11) befestigt ist, und eine Führungsnut (35) enthält, die längs des Beines (18) ausgebildet ist, welcher Stift (34) und welche Nut (35) mit relativem Verschieben gekoppelt sind.

4. Fernbediente endoskopische Kapsel nach einem der vorhergehenden Ansprüche, **dadurch gekennzeichnet, dass** sie eine erste Gruppe (20) von Beinen (18), die nahe des Vorderteils (12) des Körpers (11) angeordnet sind, und eine zweite Gruppe (21) von Beinen (18) enthält, die nahe des Hinterteils (13) des Körpers (11) positioniert sind.

5. Fernbediente endoskopische Kapsel nach einem der vorhergehenden Ansprüche, **dadurch gekennzeichnet, dass** die Schneckenwellen (24) der Antriebsvorrichtungen (22) koaxsial sind.

6. Fernbediente endoskopische Kapsel nach einem der vorhergehenden Ansprüche, **dadurch gekennzeichnet, dass** jeder Motor (23) mit der entsprechenden Schneckenwelle (24) mittels eines Getriebes verbunden ist, das ein Übertragungsverhältnis von geringer als 1 hat.

7. Fernbediente endoskopische Kapsel nach den Ansprüchen 4, 5 oder 6, **dadurch gekennzeichnet, dass** das Getriebe ein erstes Getrieberad (28), das an dem Ende der Antriebswelle (27) des Motors (23) befestigt ist, und ein einen größeren Durchmesser als das erste Getrieberad (28) aufweisendes zweites Getrieberad (29) enthält, das an dem Ende der Schneckenwelle (24) befestigt ist, welche zwei Motoren (23) von den zwei Antriebsvorrichtungen (22) der zwei Gruppen (20, 21) von Beinen (18) an entgegengesetzten Seiten der Schneckenwellen (24) positioniert sind.

8. Fernbediente endoskopische Kapsel nach Anspruch 7, **dadurch gekennzeichnet, dass** das Übertragungsverhältnis des Getriebes zwischen 0,420 und 0,430 ist.

9. Fernbediente endoskopische Kapsel nach einem der vorhergehenden Ansprüche, **dadurch gekennzeichnet, dass** der Körper (11) mit länglichen Durchgangsschlitzen (37) an der Außenoberfläche zum Unterbringen der Beine (18) darin ausgebildet ist.

10. Fernbediente endoskopische Kapsel nach Anspruch 9, **dadurch gekennzeichnet, dass** zwei Gruppen von winkelmäßig gleich beabstandeten Schlitzen (37), mit einer für jede Gruppe (20, 21) von Beinen (18), entsprechend von dem vorderen Ende und von dem hinteren Ende des Körpers (11) bis zu einer Zwischenposition verlaufen, wobei die Schlitze von einer Gruppe und die Schlitze von der anderen Gruppe in in Zweiergruppen versetzten Positionen eingestellt sind.

11. Fernbediente endoskopische Kapsel nach einem der vorhergehenden Ansprüche, **dadurch gekennzeichnet, dass** der Vorderteil (12) des Körpers (11) mit einer transparenten Kuppel (14) ausgestattet ist, innerhalb welcher die Bilderfassungseinrichtungen (15), insbesondere eine Videokamera, angebracht sind.

12. Fernbediente endoskopische Kapsel nach einem der vorhergehenden Ansprüche, **dadurch gekennzeichnet, dass** wenigstens ein Motor (23) ein bürstenloser Gleichstromelektromotor ist, der einen Außendurchmesser, der zwischen 3,5 mm und 4 mm enthalten ist, und eine Gesamtlänge hat, die zwischen 15,2 mm und 17,2 mm enthalten ist, welcher Motor mit einem Drehzahlminderer ausgestattet ist, wobei das maximale Drehmoment, das an die Welle des Drehzahlminderers abgegeben wird, gleich ist zu ungefähr 2,92 mNm.

13. Fernbediente endoskopische Kapsel nach einem der vorhergehenden Ansprüche, **dadurch gekennzeichnet, dass** jede Gruppe (20, 21) von Beinen (18) durch eine Anzahl von Beinen (18) zwischen vier und sechs gebildet ist, wobei die Vorsprünge der freien Enden (38) der Beine (18) auf einer Ebene orthogonal zu der Achse der Schneckenwellen (24) im wesentlichen auf demselben Umfang liegen, dessen Zentrum mit der Achse zusammenfällt, wobei die freien Enden (36) im wesentlichen gleich beabstandet voneinander längs des Umfangs sind, wobei die Bewegungseinrichtungen (19) die Anordnung der Beine (18) von einer maximal abgespreizten Ausdehnung, in der die Beine (18) über das Äußere des Körpers (11) auswärts vorstehen, bis zu einer minimal abgespreizten Ausdehnung zulassen, in der die Beine (18) längs des Körpers (11) positioniert sind.

14. Fernbediente endoskopische Kapsel nach Anspruch 13, **dadurch gekennzeichnet, dass** die Beine (18) von beiden Gruppen (20, 21) in der maximal ausgespreizten Ausdehnung positioniert sind, wobei die Vorsprünge der freien Enden (36', 36") von verschiedenen Gruppen (20, 21) auf der Ebene orthogonal zu der Achse der entsprechenden Schneckenwellen (24) abgewechselnd und im wesentlichen gleich beabstandet längs desselben Umfangs sind.

15. Fernbediente endoskopische Kapsel nach einem der Ansprüche 3 bis 14, **dadurch gekennzeichnet, dass** jedes Bein (18) an einem Zwischenpunkt zwischen dem freien Ende (36) und der Führungsnut (35) einen elastischen Knieteil (38) bietet, der einen weiteren Freiheitsgrad bereitstellt, um das Bein an die nachgiebige Natur des Gewebes anzupassen, mit welchem es in Kontakt kommt.

16. Fernbediente endoskopische Kapsel nach Anspruch 15, **dadurch gekennzeichnet, dass** zwei entgegengesetzte Verlängerungen (39) an dem Knieteil (38) vorgesehen sind, um aneinander anzustoßen, um die Drehung des Beins (18) in der Richtung seiner Ausdehnung zu begrenzen.

17. Fernbediente endoskopische Kapsel nach Anspruch 15 oder 16, **dadurch gekennzeichnet, dass** ein weiteres Paar von Verlängerungen (40) an dem Knieteil (38) vorgesehen ist, um nach einer weiten Drehung um den Knieteil (38) aneinander anzustoßen.

18. Fernbediente endoskopische Kapsel nach einem der vorhergehenden Ansprüche, **dadurch gekennzeichnet, dass** das freie Ende (36) jedes Beins (18) eine Hakenform hat.

19. Fernbediente endoskopische Kapsel nach einem der vorhergehenden Ansprüche, **dadurch gekennzeichnet, dass** sie eine Gesamtlänge, die im wesentlichen zwischen 24 mm und 28 mm enthalten ist, einen Öffnungswinkel der Beine, der im wesentlichen zwischen 90° und 130° enthalten ist, elektrische Motoren, die nicht mehr als 10,5 % des Gesamtvolumens des Körpers (11) belegen und eine an den freien Enden (36) jedes der Beine (18) erzeugte Kraft hat, die im wesentlichen zwischen 1,8 N und 3,2 N ist.

## Revendications

1. Capsule endoscopique télécommandée à but de diagnostics et/ou de thérapies à l'intérieur d'une cavité d'un corps humain, comportant un corps (11) ayant une partie avant (12) et une partie arrière (13), des pattes de déplacement (18) aptes à s'étendre à partir du dit corps (11) et des moyens (19) pour bouger lesdites pattes (18) logés dans le même corps (11), une source d'énergie (16), des moyens d'acquisition d'images (15), des moyens de réception/transmission de signaux (17) à partir de et vers un opérateur pour permettre la commande de la capsule et le transfert d'images acquises, où lesdites pattes (18) sont articulées sur ledit corps (11) et sont subdivisées en deux groupes séparés (20, 21), **caractérisée en ce que** les moyens pour bouger lesdites pattes (19) comportent deux dispositifs d'entraînement (22) chacun comportant un moteur (23) relié à une vis sans fin correspondante (24) avec laquelle coopère un curseur à écrou de vis pouvant être translaté (25), ledit écrou de vis étant cinématiquement relié aux pattes (18) d'un groupe respectif (20, 21).

2. Capsule endoscopique télécommandée selon la revendication 1, **caractérisée en ce que** ledit au moins un premier groupe (20) comporte, à une première extrémité de chaque patte (18), une première butée (30) constituée par une charnière, pour fixer de façon pivotante la patte sur le curseur à écrou de vis approprié (25) et, en position intermédiaire de la même patte (18), une seconde butée (32) pour permettre une roto-translation, avec l'axe de rotation parallèle à l'axe de ladite charnière (31), par rapport à un point fixe (33) du dit corps (11).

3. Capsule endoscopique télécommandée selon la revendication 2, **caractérisée en ce que** ladite seconde butée (32) comporte une goupille de rotation (34) fixée au dit corps (11) et une rainure de guidage (35) formée le long de ladite patte (18), ladite goupille (34) et ladite rainure (35) étant couplées en glissement relatif.

4. Capsule endoscopique télécommandée selon l'une quelconque des revendications précédentes, **caractérisée en ce qu'**elle comprend un premier groupe (20) de pattes (18) agencé à proximité de la partie avant (12) du dit corps (11) et un second groupe (21) de pattes (18) positionné à proximité de la partie arrière (13) du dit corps (11).

5. Capsule endoscopique télécommandée selon l'une quelconque des revendications précédentes, **caractérisée en ce que** les vis sans fin (24) des dits dispositifs d'entraînement (22) sont coaxiales.

6. Capsule endoscopique télécommandée selon l'une quelconque des revendications précédentes, **caractérisée en ce que** chaque moteur (23) est relié à la vis sans fin correspondante (24) à l'aide d'un embrayage ayant un rapport de transmission inférieur à 1.

7. Capsule endoscopique télécommandée selon la revendication 6, **caractérisée en ce que** ledit embrayage comporte une première roue dentée (28) fixée à l'extrémité de l'arbre d'entraînement (27) du dit moteur (23) et une seconde roue dentée (29), ayant un diamètre supérieur à celui de la première roue dentée (28), fixée à l'extrémité de ladite vis sans fin (24), lesdits deux moteurs (23) des deux dits dispositifs d'entraînement (22) des deux groupes (20, 21) de pattes (18) étant positionnés de part et d'autre des dites vis sans fin (24).

8. Capsule endoscopique télécommandée selon la revendication 7, **caractérisée en ce que** le rapport de transmission du dit embrayage est compris entre 0,420 et 0,430.

9. Capsule endoscopique télécommandée selon l'une quelconque des revendications précédentes, **caractérisée en ce que** ledit corps (11) comporte des fentes traversantes longitudinales (37) sur la surface extérieure pour y loger lesdites pattes (18).

10. Capsule endoscopique télécommandée selon la revendication 9, **caractérisée en ce que** deux groupes de fentes angulairement équidistantes (37), un pour chaque groupe (20, 21) de pattes (18), s'étendent respectivement à partir de l'extrémité avant et à partir de l'extrémité arrière du dit corps (11) jusqu'à une position intermédiaire, les fentes d'un groupe et les fentes de l'autre groupe étant agencées deux par deux dans des positions décalées.

11. Capsule endoscopique télécommandée selon l'une quelconque des revendications précédentes, **caractérisée en ce que** la partie avant (12) du dit corps (11) est équipée d'un dôme transparent (14) à l'intérieur duquel sont montés les moyens d'acquisition d'images (15), en particulier une caméra vidéo.

12. Capsule endoscopique télécommandée selon l'une quelconque des revendications précédentes, **caractérisée en ce que** ledit au moins un moteur (23) est un moteur électrique à courant continu, un moteur électrique sans balais, ayant un diamètre extérieur compris entre 3,5 mm et 4,5 mm et une longueur totale comprise entre 15,2 mm et 17,2 mm, ledit moteur étant équipé d'un réducteur de vitesse, le couple maximum délivré sur l'arbre du dit réducteur de vitesse étant approximativement égal 2,92 mNm.

13. Capsule endoscopique télécommandée selon l'une quelconque des revendications précédentes, **caractérisée en ce que** chaque groupe (20, 21) de pattes (18) est formé d'un nombre de pattes (18) compris entre quatre et six, les projections des extrémités libres (36) des dites pattes (18) dans un plan orthogonal à l'axe des dites vis sans fin (24) se trouvant sensiblement sur une même circonférence dont le centre coïncide avec ledit axe, lesdites extrémités libres (36) étant sensiblement équidistantes entre elles le long de ladite circonférence, lesdits moyens (19) pour bouger les pattes permettant l'agencement des dites pattes (18) à partir d'une extension déployée maximum où lesdites pattes (18) se projettent à l'extérieur du dit corps (11) jusqu'à une extension déployée minimum où lesdites pattes (18) sont positionnées le long du dit corps (11).

14. Capsule endoscopique télécommandée selon la revendication 13, **caractérisée en ce que**, lesdites pattes (18) des deux groupes (20, 21) étant positionnées en extension déployée maximum, les projections des extrémités libres (36', 36") des différents groupes (20, 21) sur le plan orthogonal à l'axe des vis sans fin correspondantes (24) sont alternées et sensiblement équidistantes le long d'une même circonférence.

15. Capsule endoscopique télécommandée selon l'une quelconque des revendications 3 à 14, **caractérisée en ce que** chaque patte (18) présente, en un point intermédiaire entre ladite extrémité libre (36) et ladite rainure de guidage (35), une partie articulée élastique (38) qui donne un degré de liberté supplémentaire pour adapter la patte à la résistance du tissu avec lequel elle est en contact.

16. Capsule endoscopique télécommandée selon la revendication 15, **caractérisée en ce que** deux prolongements en opposition (39) sont prévus sur ladite partie articulée élastique (38) aptes à buter l'un contre l'autre pour limiter la rotation de ladite patte (18) dans la direction de son extension.

17. Capsule endoscopique télécommandée selon l'une des revendications 15 et 16, **caractérisée en ce qu'**une autre paire de prolongements (40) est prévue sur ladite partie articulée élastique (38) aptes à buter l'un contre l'autre après une large rotation autour de ladite partie articulée (38).

18. Capsule endoscopique télécommandée selon l'une quelconque des revendications précédentes, **caractérisée en ce que** l'extrémité libre (36) de chaque patte (18) a la forme d'un crochet.

19. Capsule endoscopique télécommandée selon l'une quelconque des revendications précédentes, **caractérisée en ce qu'**elle a une longueur hors tout sensiblement comprise entre 24 mm et 28 mm, une ouverture angulaire des pattes sensiblement comprise entre 90° et 130°, des moteurs électriques qui n'occupent pas plus de 10,5% du volume total du corps (11), et une force générée aux extrémités libres (36) du chacune des pattes (18) sensiblement comprise entre 1,8N et 3,2N.
